# EUROPÄISCHE PATENTSCHRIFT

(11) **EP 0 420 795 B1**
(45) Veröffentlichungstag und Bekanntmachung des Hinweises auf die Patenterteilung: **19.05.1993**
(21) Anmeldenummer: 90810649.5
(22) Anmeldetag: 29.08.1990
(51) Int. Cl.: A61F 2/34

(54) **Zementfrei im Becken zu verankernde Hüftgelenkspfanne**
Hip-joint cup able to be anchored in the pelvis without cement
Cavité articulaire de la hanche se fixant sans ciment dans le bassin

(30) Priorität: 28.09.1989 CH 3524/89
(43) Veröffentlichungstag der Anmeldung: 03.04.1991
(73) Patentinhaber: GEBRÜDER SULZER AKTIENGESELLSCHAFT, CH-8401 Winterthur (CH); PROTEK AG, 3110 Münsingen-Bern (CH)
(72) Erfinder: Marchetti, Pier Giorgio, Prof. Dr.-med., I-40000 Bologna (IT); Koch, Rudolf, CH-8267 Berlingen (CH); Frick, Willi, Dr., CH-3084 Wabern (CH)

(56) Entgegenhaltungen:
- EP-A- 0 121 002
- EP-A- 0 212 087
- EP-A- 0 353 171
- WO-A-85/02535
- DE-A- 3 205 526

## Beschreibung

Die Erfindung handelt von einer zementfrei im Becken zu verankernden Hüftgelenkspfanne, die aus einer metallischen Stützschale, die mehrere dornähnliche Verankerungselemente zur Verankerung im Knochengewebe aufweist und die an ihrer Aussenfläche mit einem mehrlagigen Metallgitter zur Förderung des Einwachsens belegt ist, und aus einer inneren Kunststoffschale besteht, die in der Stützschale mit einer Schnappverriegelung verankert ist. Eine solche Hüftgelenkspfanne ist aus der FR-A- 2519545 bekannt.

EP-A- 0212 087 zeigt eine Kunststoffpfanne, die an ihrer Aussenfläche Metallgitter und dornähnliche metallische Zapfen aufweist. DE-A-32 05 526 enthält eine Schablone zum Plazieren von Aufnahmelöchern im Hüftgelenkbereich und den zugehörigen Korb.

Bei Hüftgelenkpfannen besteht die allgemeine Aufgabe, eine gute Verankerung im Knochengewebe des Beckenknochens zu erreichen; einmal primär zur sofortigen Belastung und zum anderen sekundär durch Einwachsenlassen von Knochengewebe.

Es ist Aufgabe der Erfindung, ein Implantat zu schaffen, dessen Stützschale mit dem Einpressen im Knochengewebe eine Lagefixierung und Verankerung bewirkt und eine zusätzliche Verstärkung der Verankerung ermöglicht. Gemäss der Erfindung wird die Aufgabe gelöst, indem die Stützschale an ihrer Außenfläche mit einem mehlagigen Metallgitter zur Förderung des Einwachsens belagt ist und aus tiefgezogenem Blech besteht, das zwei auf einer Schalenhälfte nach Aussen gerichtete kragenförmige Durchrisse aufweist, die vorstehende sich verjüngende Schneidkanten besitzen und die sich von der jeweiligen Schneidkante nach Innen konisch erweitern. Der Vorteil der Erfindung ist darin zu sehen, dass mit dem Einpressen der kragenförmigen Durchrisse Zapfen aus Knochengewebe stehen bleiben, die das Einwachsen im Innenraum vom Durchriss in Form eines sich erweiternden und mechanisch sichernden Pfropfens fördern oder dass die Durchrisse nach dem Einpressen als Bohrführung dienen, um zusätzliche Verankerungsschrauben aufzunehmen. Nach dem Einpressen der Stützschale kann auf Grund vom Zustand d.h. der Haltewirkung des Knochengewebes entschieden werden, ob weitere Verankerungselemente notwendig sind, die ohne grosse Vorbereitung angebracht werden können. Ein weiterer Vorteil besteht in der fertigungstechnischen Gestaltung der Stützschale, die in Leichtbauweise auf Grossserien abgestimmt ist, indem als Ausgangsmaterial ein verformbares Blech eingesetzt wird.

Im folgenden wird die Erfindung anhand von einem Ausführungsbeispiel beschrieben. Es zeigen:

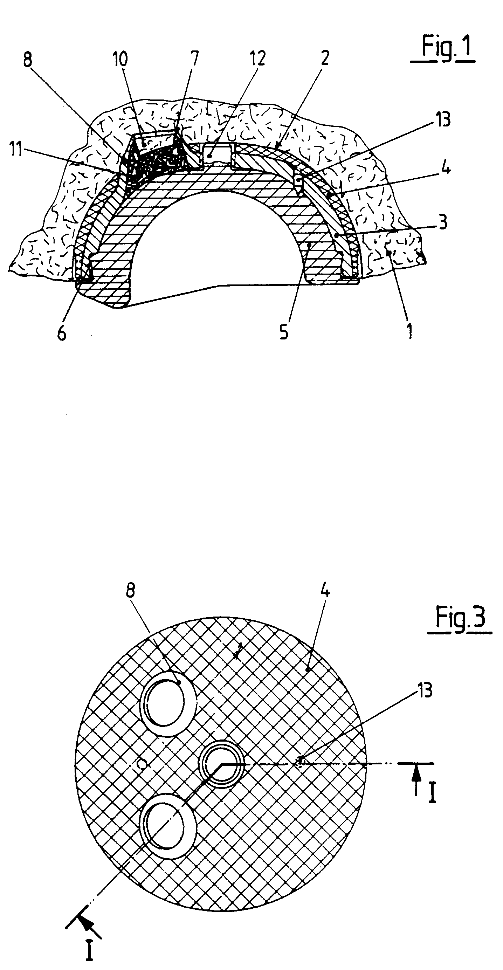

- Fig. 1: die Seitenansicht eines Schnittes durch eine Hüftgelenkpfanne nach dem Implantieren im Knochengewebe,

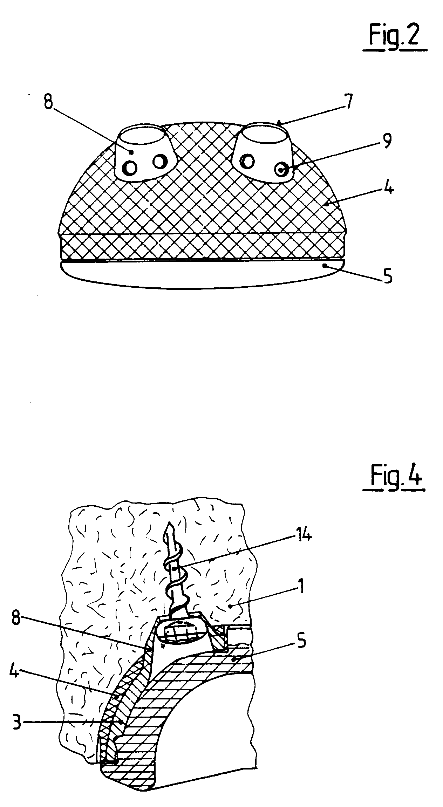

- Fig. 2: eine Seitenansicht einer Hüftgelenkpfanne,
- Fig. 3: eine Draufsicht einer Hüftgelenkpfanne und
- Fig. 4: den Ausschnitt einer Seitenansicht wie in Fig. 1 mit einer zusätzlichen Verankerung.

In den Figuren ist eine zementfreie im Becken zu verankernde Hüftgelenkpfanne gezeigt, bestehend aus einer metallischen Stützschale 3, die mehrere dornähnliche Verankerungselemente 8 zur Verankerung im Knochengewebe 1 durch Einpressen aufweist und die an ihrer Aussenfläche mit einem mehrlagigen Metallgitter 4 zur Förderung des Einwachsens belegt ist, und aus einer Kunststoffschale 5, die in der Stützschale mit einer Schnappverriegelung 6 verankert ist.

Die leere Stützschale 3 wird in eine kalottenförmige im Beckenknochen vorbereitete Aushöhlung 2 eingepresst. Erfindungsgemäss besteht die Stützschale 3 aus tiefgezogenem Blech, das zwei auf einer Schalenhälfte nach aussen gerichtete kragenförmige Durchrisse 8 aufweist, die vorstehende sich verjüngernde Schneidkanten 7 besitzen und die sich von der jeweiligen Schneidkante nach Innen konisch erweitern. Beim Einpressen graben sich die Durchrisse 8 in das Knochengewebe 1 ein und bilden mit ihrer Aussenfläche eine Verankerung gegen Verrutschen der Stützschale 3. Gleichzeitig bleibt auf der Innenseite ein Knochenzapfen 10 stehen. Die eigentliche Verankerung bildet der hinterschnittene Aussenrand der Stützschale, an dem das Metallgitter 4 zurückgesetzt ist und vom Knochengewebe 1 gehalten wird. Bei genügender Verankerung der Stützschale 3 wird der Raum um die Knochenzapfen 10 mit Knochenspänen und Granulat aus Hydroxylapatit gestopft und anschliessend die Kunststoffschale 5 mit ihrer Schnappverriegelung 6 eingesetzt. Bei ungenügender Verankerung der Stützschale 3 wird der Zapfen 10 entfernt und eine Knochenschraube 14 gesetzt, die mit ihrem Kopf am Innenkonus vom Durchriss 8 aufliegt und die Stützschale 3 sichert. Eine Grobzentrierung der Kunststoffschale wird mit dem Zapfen 12 vorgenommen, während mit den Stiften 13 die Verdrehung zwischen Stützschale 3 und Kunststoffschale 5 nach dem Einrasten der Schnappverriegelung 6 verhindert wird.

Zum besseren Einwachsen der Knochenzapfen 10 sind Oeffnungen 9 im Durchriss 8 vorgesehen.

## Patentansprüche

1. Zementfrei im Becken zu verankernde Hüftgelenkspfanne bestehend aus einer metallischen Stützschale (3), die mehrere dornähnliche Verankerungselemente zur Verankerung im Knochengewebe durch Einpressen aufweist, und aus einer inneren Kunststoffschale (5), die in der Stützschale (3) mit einer Schnappverriegelung (6) verankert ist, dadurch **gekennzeichnet**, dass die Stützschale (3) an ihrer Aussenfläche mit einem mehrlagigen Metallgitter (4) zur Förderung des Einwachsens belegt ist und aus tiefgezogenem Blech besteht, das zwei auf einer Schalenhälfte nach aussen gerichtete kragenförmige Durchrisse (8) aufweist, die vorstehende sich verjüngende Schneidkanten (7) besitzen und die sich von der jeweiligen Schneidkante nach innen konisch erweitern.

2. Hüftgelenkspfanne nach Anspruch 1, dadurch gekennzeichnet, dass die Schnappverriegelung (6) ein Einsetzen der Kunststoffschale (5) nach dem Einpressen der Stützschale (3) in einer Aushöhlung (2) des Knochengewebes (1) ermöglicht.

3. Hüftgelenkspfanne nach Anspruch 1 und 2, dadurch gekennzeichnet, dass der konische Teil der Durchrisse (8) Ausnehmungen (9) aufweist.

## Claims

1. An acetabulum to be secured in the pelvis without the use of cement, which consists of a metal suporting shell (3), which comprises several plug-like retaining elements to be secured in the osseous tissue by being forced in, and of an inner plastic shell (5), which is secured in the supporting shell (3) with a spring catch (6),
**characterised in that** the supporting shell (3) is covered on its outer surface with a multi-layer metal mesh (4) for promoting fusion and is made from deep-drawn sheet metal, which comprises two collar-shaped tears (8) outwardly directed on one shell half, which have projecting, tapering cutting edges (7) and which extend inwardly from the respective cutting edge in a conical shape.

2. An acetabulum according to Claim 1,
**characterised in that** the spring catch (6) enables the plastic shell (5) to be inserted after the supporting shell (3) has been forced into a groove (2) in the osseous tissue (1) .

3. An acetabulum according to Claim 1 and 2,
**characterised in that** the conical part of the tears (8) comprises clearances (9).

## Revendications

1. Cavité cotyloïde se fixant sans ciment dans le bassin et se composant d'une coque métallique de soutien (3) comportant plusieurs éléments en forme de mandrins d'ancrage par enfoncement dans le tissu osseux, ainsi que d'une coque intérieure (5) en matière plastique qui s'ancre dans la coque de soutien (3) par un verrouillage (6) à enclenchement élastique, caractérisée en ce que la coque de soutien (3) est revêtue sur la surface extérieure d'une toile métallique (4) en plusieurs couches destinée à favoriser la croissance et consiste en une tôle ayant subi un emboutissage profond et comportant sur une moitié deux repoussages ouverts (8) en forme de collets orientés vers l'extérieur, qui comportent des arêtes saillantes de coupe (7) allant en se rétrécissant et qui s'élargissent en cône vers l'intérieur à partir desdites arêtes de coupe.

2. Cavité cotyloïde selon la revendication 1, caractérisée en ce que le verrouillage (6) à enclenchement élastique permet la mise en place de la coque (5) en matière plastique après enfoncement de la coque de soutien (3) dans un évidement (2) du tissu osseux (1).

3. Cavité cotyloïde selon les revendications 1 et 2, caractérisée en ce que la partie conique des repoussages ouverts (8) comporte des trous (9).
